## Europäisches Patentamt

## European Patent Office

## Office européen des brevets

(19)

(11) Veröffentlichungsnummer: **0 039 323**
**B1**

(12) 

# EUROPÄISCHE PATENTSCHRIFT

(45) Veröffentlichungstag der Patentschrift:
22.02.84

(51) Int. Cl.³: **A 61 F 5/04**

(21) Anmeldenummer: **81890067.2**

(22) Anmeldetag: **16.04.81**

(54) Schiene zur Reposition und Ruhigstellung von Finger- und Mittelhandfrakturen, sowie Verfahren zur Herstellung derselben.

(30) Priorität: **25.04.80 AT 2252/80**

(43) Veröffentlichungstag der Anmeldung:
**04.11.81 Patentblatt 81/44**

(45) Bekanntmachung des Hinweises auf die Patenterteilung:
**22.02.84 Patentblatt 84/8**

(84) Benannte Vertragsstaaten:
**BE DE FR GB NL**

(56) Entgegenhaltungen:
**CH - A - 253 834**
**GB - A - 861 081**
**US - A - 2 980 110**
**US - A - 3 804 085**
**US - A - 4 143 653**

(73) Patentinhaber: **Ender, Hans Georg, Dr.,
Ferstelgasse 6/20, A-1090 Wien (AT)**

(72) Erfinder: **Ender, Hans Georg, Dr., Ferstelgasse 6/20,
A-1090 Wien (AT)**

(74) Vertreter: **Boeckmann, Peter, Dipl.Ing. et al,
Strohgasse 10, A-1030 Wien (AT)**

BUNDESDRUCKEREI BERLIN

## Schiene zur Reposition und Ruhigstellung von Finger- und Mittelhandfrakturen, sowie Verfahren zur Herstellung derselben

Die Erfindung betrifft eine Schiene zur Reposition und Ruhigstellung von Finger- und Mittelhandfrakturen, bestehend aus einem plastisch verformbaren Streifen, der an seinem einen Endbereich zumindest einen Querteil zur Fixierung am Vorderarm aufweist.

An eine solche Schiene werden die Anforderungen gestellt, daß eine Anpassung an den gebrochenen Finger- bzw. die gebrochene Mittelhand durchführbar sein muß, daß jedoch die angepaßte Form dann beibehalten werden muß, damit eine einwandfreie Abstützung des Bruches gewährleistet ist.

Man hat bereits vorgeschlagen, derartige Schienen aus einem thermoplastischen Kunststoff herzustellen, der bei Körpertemperatur starr ist, jedoch bei einer höheren Temperatur erweicht und dadurch verformbar wird. Die Anpassung derartiger Schienen ist jedoch umständlich und zeitraubend, da ein dauerndes Erwärmen und Abkühlen des Kunststoffes erforderlich ist, bis die Schiene die erforderliche Gestalt besitzt.

Es ist auch bereits eine Schiene bekannt, die aus einem Drahtgerüst besteht, das mit Verbandstoff od. dgl. umwickelt ist. Ein solches Drahtgerüst kann zwar leicht in die gewünschte Form gebogen werden, es besteht jedoch die Gefahr, daß beim Biegen das Drahtgerüst derart deformiert wird, daß keine optimale Auflagefläche für den Finger gegeben ist. Außerdem ist bei dieser Ausführungsform nicht sichergestellt, daß das Drahtgerüst in der Folge seine dem Bruch angepaßte Form beibehält, wie dies für den Heilungsprozeß erforderlich ist.

Eine andere bekannte Fingerschiene besteht aus einer zylindrischen Hülse, die über den eine Fraktur aufweisenden Finger geschoben wird und in der der gestreckte Finger ruhiggestellt wird. Diese Hülse weist quer zu ihrer Längsrichtung verlaufende Schlitze auf, in welche federnde Drahtringe eingesetzt werden mittels welcher die Hülse fixiert wird. Außerdem besitzt die Hülse Öffnungen, welche den Luftzutritt zum Finger ermöglichen und über welche auch Wasser ein- und austreten kann. Eine solche Anordnung ermöglicht einerseits nur die Abstützung des äußeren Gliedes und des Mittelgliedes eines Fingers, ist somit nur in beschränktem Umfang anwendbar, und läßt eine Fixierung des Fingers in einer mittleren Beugestellung nicht zu, wie dies aus später noch ausführlich erläuterten Gründen in der Regel erforderlich ist.

Eine in der US-A-4 143 653 geoffenbarte Fingerschiene besteht aus einem Streifen, von dem an einer Seite ein Fortsatz vorsteht, so daß eine Abstützung des Fingers durch zwei zueinander im wesentlichen senkrecht verlaufende Flächen erfolgt. Um den Streifen in eine dem Finger angepaßte konkave Form biegen zu können, sind im Fortsatz Schlitze vorgesehen, die es ermöglichen, daß der Fortsatz der Form des gebogenen Streifens zu folgen vermag. Auch bei dieser Ausführungsform kann jedoch der Streifen nur senkrecht zu seiner Ebene gekrümmt sowie gegebenenfalls um seine Längsachse verdreht werden.

Die vorliegende Erfindung hat sich zur Aufgabe gestellt, diese Nachteile zu vermeiden und eine Schiene zur Reposition und Ruhigstellung von Finger- und Mittelhandfrakturen zu schaffen, die leicht in die anatomisch richtige Form gebracht werden kann, jedoch die gewünschte Form beibehält. Soll der Streifen in die anatomisch richtige Form gebracht werden, so ist zu berücksichtigen, daß die Achsen der Finger von einer quer verlaufenden Handwölbung ausgehen und beim Abbiegen der Finger nicht mehr parallel verlaufen wie bei der Streckstellung, sondern am Kahnbeinhöker zusammenlaufen. Die Fingerachsen verändern also von einer Parallelstellung in der Streckstellung der Finger ausgehend während des Abbiegens der Finger dauernd ihre Richtung, bis sie letztlich bei vollständig abgebogenen Fingern auf den Kahnbeinhöker zielen. Eine Ruhigstellung der eine Fraktur aufweisenden Finger kann nun nicht in der gestreckten Lage der Finger erfolgen, sondern muß in einer mittleren Beugestellung der Finger vorgenommen werden, damit die Sehnen gestreckt sind und die Finger nicht versteifen. Dies bedingt, daß auch die Fingerschiene in eine der jeweiligen mittleren Beugestellung des ruhigzustellenden Fingers entsprechende Lage gebracht werden muß. Bei bekannten Schienen ist es nur möglich, eine Verdrehung quer zu ihrer Längsachse vorzunehmen, so daß die Richtung der Schienenachse in der Regel nicht mehr mit der Achse des in mittlerer Beugestellung befindlichen Fingers übereinstimmt. Dadurch erfolgt eine unerwünschte Verdrehung des gebrochenen, durch die Schiene geschienten Fingers, so daß die richtige Ruhigstellung des durch die Schiene unterstützten Fingers nicht gewährleistet ist.

Die Erfindung vermeidet nun diesen Nachteil dadurch, daß, ausgehend von einer Schiene der eingangs beschriebenen Art, der Streifen an seinen beiden Längsrändern mit Schwächungen versehen ist, welche ein Verbiegen des Streifens in seiner Ebene ermöglichen, und daß der Streifen vorzugsweise zur Gänze mit einer Umhüllung aus nachgiebigem Material umgeben ist. Die Schwächungen des Streifens an seinen beiden Längsrändern ermöglichen es, daß der Streifen nicht nur in seiner Ebene verbogen und um seine Längsachse verdreht werden kann, sondern auch quer zur Längsachse in seiner Ebene verbogen werden kann, so daß dann die Längsachse eine in zwei im wesentlichen zueinander verdrehten Ebenen gekrümmten Kurve darstellt. Dadurch wird sichergestellt, daß die Schiene für jeden Finger und jeden Bruch exakt in eine Lage gebracht werden kann, die der anatomischen Form und Lage des Fingers angepaßt ist.

Die Schwächungen können gemäß einem weiteren Merkmal der Erfindung beispielsweise dadurch gebildet sein, daß der Streifen an seinen beiden Längsrändern Ausnehmungen aufweist, welche aus geradlinig begrenzten Zwickeln gebildet sein können oder von einer Kurve begrenzt sein können.

Die Umhüllung ist zweckmäßig aus einem verformbaren Kunststoff, beispielsweise aus Polyurethanschaum gebildet.

Um nun einerseits die Schiene exakt in die richtige Lage zu bringen, also von einer Streckstellung des an der Schiene befestigten Fingers ausgehend so zu verbiegen, daß dieser in eine mittlere Beugestellung gelangt und dabei die Bruchstelle reponiert wird, und andererseits sicherzustellen, daß die Schiene in dieser Lage verbleibt und nicht wieder durch den Patienten aufgebogen wird, kann gemäß einem weiteren Merkmal der Erfindung zwischen den beiden Endbereichen des Streifens eine längenveränderbare Fixiervorrichtung vorgesehen sein. Durch Verkürzung der Länge dieser Fixiervorrichtung wird die Schiene verbogen und dadurch in eine Lage gebracht, die der erforderlichen mittleren Beugestellung des Fingers entspricht, und es wird nach dem Festlegen der Fixiervorrichtung in dieser Lage der Schiene ein Aufbiegen derselben verhindert.

Die Fixiervorrichtung besteht hierbei zweckmäßig aus einem drahtförmigen oder streifenbzw. bandförmigen Material, beispielsweise aus einem selbstklebenden Streifen, der zunächst am fingerspitzenseitigen Endbereich der Schiene und nach dem Verbiegen derselben am gegenüberliegenden Endbereich befestigt wird. Es kann aber auch die Fixiervorrichtung an wenigstens einem Ende mit einer selbstsperrenden Klemmvorrichtung versehen sein, also beispielsweise mit an einer Seite abgeschrägten Zähnen, die sich durch eine Öse od. dgl. in einer Richtung durchziehen lassen, sich bei einem Zug in der anderen Richtung jedoch in der Öse verklemmen. Solche selbstsperrende Klemmvorrichtungen sind an sich bekannt.

Die erfindungsgemäße Schiene kann auch leicht hergestellt werden. Nach einem erfindungsgemäßen Verfahren zur Herstellung der Schiene wird zunächst der Streifen mit seinen Querteilen und den Schwächungen bzw. Ausnehmungen, beispielsweise durch Stanzen aus einer Blechtafel, gebildet und anschließend in eine Form eingelegt, worauf diese geschlossen und mit Kunststoffmaterial ausgegossen oder ausgespritzt wird. Nach einem anderen erfindungsgemäßen Verfahren zur Herstellung der Schiene wird zunächst der Streifen mit seinen Querteilen und seinen Schwächungen bzw. Ausnehmungen, beispielsweise durch Stanzen aus einer Blechtafel, gebildet und anschließend zwischen zwei Lagen aus die Umhüllung bildendem, vorzugsweise textilen, Material eingelegt, wobei die beiden Lagen am Streifenrand miteinander verbunden werden, und es wird der überschüssige Teil des die Umhüllung bildenden Materials entsprechend der Form der Schiene abgetrennt.

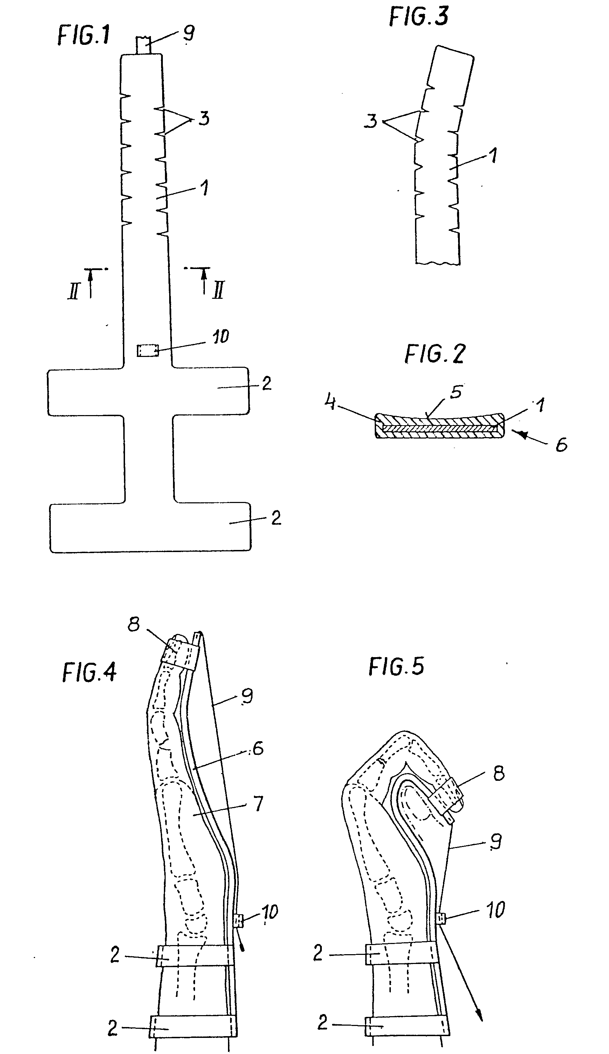

Im folgenden wird die Erfindung an Hand der Zeichnung näher erläutert. Fig. 1 zeigt schematisch einen Streifen zur Herstellung einer erfindungsgemäßen Schiene. Fig. 2 stellt einen Schnitt nach der Linie II-II in Fig. 1 dar, wobei jedoch auf dem Streifen die Umhüllung aufgebracht ist. Fig. 3 zeigt das obere Ende des Streifens nach dessen Verbiegung. Die Fig. 4 und 5 zeigen die Anwendung der erfindungsgemäßen Schiene, wobei Fig. 4 den durch die Schiene unterstützten Finger in seiner im wesentlichen gestreckten Lage und Fig. 5 in einer mittleren Beugestellung darstellt.

Der die Schiene bildende Streifen 1 besteht aus einem biegbaren Material, vorzugsweise aus einem röntgenstrahlendurchlässigen Metallblech, beispielsweise Aluminiumblech, und ist mit zwei Querteilen 2 versehen, welche einstückig mit dem Streifen 1 ausgebildet sind und welche dazu dienen, die Schiene mit ihrem einen Ende am Handgelenk zu fixieren, um welches die Querteile 2 herumgebogen werden. Anstelle von zwei Querteilen kann auch ein einziger Querteil vorgesehen sein, welcher dann vorzugsweise breiter ausgebildet ist. Es ist auch möglich, die Querteile 2 nicht einstückig mit dem Streifen 1 auszubilden, sondern gesondert herzustellen und mit dem Streifen 1 durch Verkleben, Vernieten oder Verschweißen zu verbinden.

Um nun die Bruchstelle zu reponieren und den Finger in der richtigen Lage ruhigzustellen, muß in später noch näher beschriebener Weise die Schiene in eine bestimmte Lage gebogen werden. Bisher bekannte Schienen konnten nur entweder um ihre Längsachse verdreht oder so verbogen werden, daß die Längsachse von einer Geraden in eine Kurve verbogen wird. Diese beiden Verdreh- bzw. Verbiegemöglichkeiten der Schiene genügen jedoch nicht, um diese in eine Lage zu bringen, in welcher eine optimale Unterstützung des Fingers in seiner richtigen Stellung gewährleistet ist. Der Bau der Hand und der Finger ist nämlich derart, daß die Hand ein Längs- und ein Quergewölbe darstellt. Das Längsgewölbe in Verbindung mit den Fingergelenken ermöglicht der Hand, einen beispielsweise zylinderförmigen Gegenstand zu umgreifen und zu erfassen. Das Quergewölbe dagegen modelliert die Hand auch quer und erweitert die Bewegungsmöglichkeit der Hand um eine weitere Dimension, so daß beispielsweise auch eine Kugel von der Hand umfaßt werden kann. Deshalb sind die Finger so gebaut, daß ihre Achsen zwar in der Streckstellung im wesentlichen parallel verlaufen, beim Abbiegen der Finger jedoch die nun eine Kurve darstellenden Achsen nicht mehr parallel sind und in der Beugestellung schließlich so verlaufen, daß sie alle auf einen Punkt etwa im Bereich des Daumenballens zielen. Wenn nun eine Schiene einen in der Beugestellung befindlichen Finger in optimaler Weise unterstützen soll und die Drehfehler an der Bruchstelle ausgeschlossen werden sollen, so genügt die Verdreh- und Verbiegemöglichkeit der bisher bekannten Schienen dazu nicht. Bei der erfindungsgemäßen Schiene ist nun der Streifen 1 an seinen beiden Längsrändern mit geradlinig begrenzten, die Form von Zwickeln aufweisenden Ausnehmungen 3 versehen. Anstelle der dargestellten zwickelförmigen Ausnehmungen können auch von Kreisbögen oder von anderen Kurven begrenzte Ausnehmungen vorgesehen sein. Diese Ausnehmungen 3 ermöglichen es, eine Querverbiegung der Schiene in ihrer Ebene vorzunehmen, wie dies in Fig. 3 veranschaulicht ist. Die Ausnehmungen 3 ermöglicht somit, daß die Schiene in drei verschiedenen Richtungen verbogen bzw. verdreht werden kann, so daß sie also in jede beliebige gewünschte Lage gebracht werden kann und dadurch eine optimale Reposition und eine optimale Unterstützung des reponierten Fingers gewährleistet ist.

Wie aus Fig. 2 hervorgeht, ist der Streifen 1 zur Gänze mit einer Umhüllung 4 aus nachgiebigem Material umgeben, die beispielsweise aus Kunststoff, vorzugsweise aus Polyurethanschaum besteht. Der vorher durch Stanzen hergestellte Streifen 1 wird zu diesem Zweck in eine Form eingelegt, und es wird nach dem Schließen der Form diese mit polyurethanbildendem Material ausgegossen oder ausgespritzt. Zweckmäßig ist hierbei die Form so gestaltet, daß an einer Seite der Schiene eine Mulde 5 gebildet wird, welche den Finger aufnimmt, wodurch die Unterstützung des Fingers verbessert wird. Es kann aber auch eine Umhüllung aus textilen Materialien verwendet werden.

Soll ein gebrochener Finger mittels der erfindungsgemäßen Schiene reponiert und ruhiggestellt werden, so wird zunächst die Schiene 6 mit ihren beiden Querteilen 2 am Handgelenk der betreffenden Hand befestigt und zunächst in eine Form gebogen, wie sie in Fig. 4 dargestellt ist. Dann wird der Finger 7 mit seiner Spitze am oberen Ende der Schiene mittels eines Heftpflasters 8 od. dgl. fixiert. An diesem oberen Ende der Schiene ist eine Fixiervorrichtung 9 befestigt, die aus einem Draht oder aus einem Streifen bestehen kann. Die Befestigung der Fixiervorrichtung 9 kann entweder dadurch erfolgen, daß das Ende des Drahtes oder Streifens in die aus Kunststoff bestehende Umhüllung 4 eingegossen wird, vorzugsweise nach vorheriger Fixierung am oberen Ende des Streifens 1, es kann aber auch das obere Ende der Schiene 6 mit einer Ausnehmung, einer Öse od. dgl. versehen sein, in die die Fixiervorrichtung 9 eingehängt werden kann. Schließlich kann ein selbstklebender Streifen verwendet werden, der durch Kleben an der Schiene 6 befestigt wird.

Das andere Ende der Fixiervorrichtung 9 ist zweckmäßig durch eine Hülse 10 od. dgl. hindurchgeführt, so daß dann, wenn an diesem Ende der Fixiervorrichtung 9 angezogen wird, die Schiene 6 verbogen wird.

Zur Reposition der Fraktur wird der Finger des Chirurgen gegen das Grundgelenk des zu reponierenden Fingers gedrückt und der Finger heruntergebogen, so daß durch die sich hierbei ergebende Spannung der Bruch, der vor allem dann, wenn er im Grundglied des Fingers vorhanden ist, durch den Zug der Muskulatur immer einen streckseitig offenen Winkel bildet, in die richtige Lage gebracht wird, also die beiden gebrochenen Gliederteile in die gerade Stellung zurückgebogen werden. Hierbei ist es wesentlich, daß der gebrochene Finger in eine ganz bestimmte Lage gebogen wird, in der gerade die beiden Teile des gebrochenen Gliedes ihre gerade Stellung besitzen. Um nun eine exakte, stufenlose Verbiegung des Fingers zum Reponieren zu ermöglichen, kann am freien Ende der Fixiervorrichtung 9 angezogen werden, bis das gebrochene Fingerglied seine reponierte Lage aufweist. Da der gebrochene Finger mittels des Heftpflasters 8 an der Schiene 6 befestigt ist, macht der Finger die Bewegung der Schiene mit. Gleichzeitig wird die Schiene 6 auch in eine solche Lage gebracht, daß der sich nun in der in Fig. 5 dargestellten Beugestellung befindliche Finger auf die Rauhigkeit des Kahnbeines hinzeigt. Dadurch wird der Finger in eine ideale Stellung gebracht, in der keine Verdrehung im Bruch erfolgt.

Das die Fixiervorrichtung bildende Band ist nun zweckmäßig an seinem durch die Hülse 10 hindurchgezogenen Ende mit Zähnen versehen, die auf einer Seite abgeschrägt sind, so daß sie bei einem Zug in einer Richtung durch die Hülse 10 hindurchtreten können, bei einem Zug in entgegengesetzter Richtung jedoch eine Arretierung des Bandes in der betreffenden Stellung erfolgt. Derartige selbstsperrende Klemmvorrichtungen sind bereits bekannt. Durch eine solche Ausbildung wird die Schiene mit dem darauf fixierten Finger in der gebogenen Stellung gehalten und kann ohne Lösen der Fixiervorrichtung nicht wieder aufgebogen werden.

Selbstverständlich ist auch eine andere Befestigung der Fixiervorrichtung im Bereich des unteren Endes der Schiene möglich, beispielsweise durch Verwendung des bereits erwähnten selbstklebenden Streifens, der an die Schiene 6 angeklebt wird oder dadurch, daß beispielsweise die einen Vorsprung bildende Hülse 10 von der drahtförmigen Fixiervorrichtung umschlungen wird.

## Patentansprüche

1. Schiene zur Reposition und Ruhigstellung von Finger- und Mittelhandfrakturen, bestehend aus einem plastisch verformbaren Streifen (1), der an seinem Endbereich zumindest einen Querteil (2) zur Fixierung am Vorderarm aufweist, dadurch gekennzeichnet, daß der Streifen (1) an seinen beiden Längsrändern mit Schwächungen (3) versehen ist, welche ein Verbiegen des Streifens (1) in seiner Ebene ermöglichen, und daß der Streifen (1) vorzugsweise zur Gänze mit einer Umhüllung (4) aus nachgiebigem Material umgeben ist.

2. Schiene nach Anspruch 1, dadurch gekennzeichnet, daß der Streifen (1) an seinen beiden Längsrändern Ausnehmungen (3) aufweist.

3. Schiene nach Anspruch 2, dadurch gekennzeichnet, daß die Ausnehmungen (3) von geradlinig begrenzte Zwickeln gebildet sind.

4. Schiene nach Anspruch 2, dadurch gekennzeichnet, daß die Ausnehmungen (3) von einer Kurve begrenzt sind.

5. Schiene nach Anspruch 1, dadurch gekennzeichnet, daß die Umhüllung (4) aus einem verformbaren Kunststoff gebildet ist.

6. Schiene nach einem der Ansprüche 1 bis 5, dadurch gekennzeichnet, daß zwischen den beiden Endbereichen des Streifens (1) eine längenveränderbare Fixiervorrichtung (9) vorgesehen ist.

7. Schiene nach Anspruch 6, dadurch gekennzeichnet, daß die Fixiervorrichtung (9) aus einem drahtförmigen oder streifen- bzw. bandförmigen Material, beispielsweise aus einem selbstklebenden Streifen, besteht.

8. Schiene nach Ansprüche 6 oder 7, dadurch gekennzeichnet, daß die Fixiervorrichtung (9) am wenigstens einem Ende mit einer selbstsperrenden Klemmvorrichtung (10) versehen ist.

9. Verfahren zur Herstellung einer Schiene nach einem der Ansprüche 1 bis 5, dadurch gekennzeichnet, daß zunächst der Streifen (1) mit seinen Querteilen (2) und den Schwächungen bzw. Ausnehmungen (3), beispielsweise durch Stanzen aus einer Blechtafel, gebildet und anschließend in eine der Gestalt der Schiene entsprechende Form eingelegt wird und diese geschlossen und hierauf mit dem Kunststoffmaterial ausgegossen oder ausgespritzt wird.

10. Verfahren zur Herstellung einer Schiene nach einem der Ansprüche 1 bis 5, dadurch gekennzeichnet, daß zunächst der Streifen (1) mit seinen Querteilen (2) und seinen Schwächungen bzw. Ausnehmungen (3) beispielsweise durch Stanzen aus einer Blechtafel, gebildet wird, daß anschließend der Streifen (1) zwischen zwei Lagen aus die Umhüllung (4) bildendem, vorzugsweise textilen, Material eingelegt wird, wobei die beiden Lagen am Streifenrand miteinander verbunden werden, und daß der überschüssige Teil des die Umhüllung (4) bildenden Materials entsprechend der Form der Schiene abgetrennt wird.

## Claims

1. Splint for the setting and immobilisation of finger and metacarpus fractures, consisting of a plastically deformable strip (1) having on its end portion at least one transverse member (2) for fixing to the fore-arm, characterized in that the strip (1) is provided on its both longitudinal edges with weakenings (3) which enable bending the strip (1) in its plane, and that the strip is surrounded by an envelope (4) of resilient material, preferably totaly.

2. Splint according to claim 1, characterized in that the strip (1) is provided with gaps (3) on its both longitudinal edges.

3. Splint according to claims 2, characterized in that the gaps (3) are formed of gores delimited by straight lines.

4. Splint according to claim 2, characterized in that the gaps (3) are delimited by a curve.

5. Splint according to claim 1, characterized in that the envelope (4) is formed of a deformable synthetic plastic material.

6. Splint according to any of claims 1 to 5, characterized in that a fixing means (9) of variable length is provided between the two end portions of the strip (1).

7. Splint according to claim 6, characterized in that the fixing means (9) consists of a wire-shaped or strip-or band-shaped, respectively, material, for example of a self-adhesive strip.

8. Splint according to claim 6 or 7, characterized in that the fixing means (9) is provided on at least one end with a self-locking clamping device (10).

9. Process for the manufacture of a splint according to anyone of the claims 1 to 5, characterized in that at first the strip (1) with its transverse members (2) and the weakenings or gaps (3), respectively, is formed, for example by punching of a sheet metal plate, and is then inserted into a mold corresponding to the shape of the splint and that the mold closed and thereupon filled with the synthetic plastic material by pouring or injecting.

10. Process for the manufacture of a splint according to anyone of the claims 1 to 5, characterized in that at first the strip (1) with its transverse members (2) and its weakenings or gaps (3), respectively, is formed, for example by punching of a sheet metal plate, and that then the strip (1) is put between two layers of a material, preferably textil material, forming the envelope (4), noting that the two layers are connected to each other on the strip edge, and that the exceeding part of the material forming the envelope (4) is separated according to the shape of the splint.

## Revendications

1. Eclisse pour la réduction et l'immobilisation des fractures des doigts et du métacarpe, constituée d'une bande plastiquement déformable (1) qui présente, à l'une de ses extrémités, au moins une branche transversale (2) pour la fixation à l'avant-bras, caractérisée en ce que la bande (1) est munie, sur ses deux bords longitudinaux, d'affaiblissements (3) qui permettent à la bande (1) de se tordre dans son propre plan et en ce que la bande (1) est entourée, de préférence en totalité, par une enveloppe (4) en matériau souple.

2. Eclisse selon la revendication 1, caractérisée en ce que la bande (1) comporte des entailles (3) sur ses deux bords longitudinaux.

3. Eclisse selon la revendication 2, caractérisée en ce que les entailles (3) sont formées par des coins limités par des segments de droite.

4. Eclisse selon la revendication 2, caractérisée en ce que les entailles (3) sont limitées par une courbe.

5. Eclisse selon la revendication 1, caractérisée en ce que l'enveloppe (4) est faite d'une matière synthétique déformable.

6. Eclisse selon l'une des revendications 1 à 5, caractérisée en ce qu'un dispositif de fixation (9), de longueur réglable, est disposé entre les deux extrémités de la bande (1).

7. Eclisse selon la revendication 6, caractérisée en ce que le dispositif de fixation (9) est fait d'un matériau en forme de fil ou de ruban ou en forme de bande, par exemple d'une bande autocollante.

8. Eclisse selon la revendication 6 ou 7, caractérisée en ce que le dispositif de fixation (9) est muni, à l'une au moins de ses extrémités, d'un dispositif de serrage (10) autobloquant.

9. Procédé pour la fabrication d'une éclisse selon l'une des revendications 1 à 5, caractérisé en ce que l'on forme d'abord, par exemple par estampage d'un flan de tôle, la bande (1) avec ses branches transversales (2) et ses affaiblissements ou entailles (3) et qu'ensuite on la pose dans un moule correspondant à la forme de l'éclisse et qu'on ferme ce moule, après quoi on remplit ce dernier, par coulée ou injection, avec le matériau constitutif de la matière synthétique.

10. Procédé pour la fabrication d'une éclisse selon l'une des revendications 1 à 5, caractérisé en ce que l'on forme d'abord, par exemple par estampage d'un flan de tôle, la bande (1) avec ses branches transversales (2) et ses affaiblissements ou entailles (3) et qu'ensuite on pose la bande (1) entre deux couches de la matière, de préférence textile, qui forme l'enveloppe (4), les deux couches étant reliées l'une à l'autre sur le bord de la bande, et en ce que l'on découpe la partie excédentaire de la matière formant l'enveloppe (4), selon la forme de l'éclisse.

FIG.1

FIG.3

FIG.2

FIG.4

FIG.5